# EUROPÄISCHE PATENTSCHRIFT

(11) **EP 1 488 746 B1**
(45) Veröffentlichungstag und Bekanntmachung des Hinweises auf die Patenterteilung: **07.03.2007**
(21) Anmeldenummer: 04010253.5
(22) Anmeldetag: 30.04.2004
(51) Int. Cl.: A61B 17/17, A61B 17/16

(54) **Vorrichtung zum lagerichtigen Einbringen eines Führungsdrahtes für ein Bohrwerkzeug in einen Knochen**
Device for correctly inserting a guide wire for a drill in a bone
Dispositf d'insertion correcte d'un fil guide pour un foret dans un os

(30) Priorität: 20.06.2003 DE 20309481 U
(43) Veröffentlichungstag der Anmeldung: 22.12.2004
(73) Patentinhaber: Stryker Trauma GmbH, 24232 Schönkirchen (DE)
(72) Erfinder: Zander, Nils, 24340 Eckernförde (DE)
(74) Vertreter: Kopf, Korbinian Paul

(56) Entgegenhaltungen:
- US-A- 5 489 284
- US-A- 5 601 550
- US-A- 5 624 447
- US-A- 5 951 561

## Beschreibung

Die Erfindung bezieht sich auf eine Vorrichtung zum lagerichtigen Einbringen eines Führungsdrahtes für ein Bohrwerkzeug in einen Knochen nach dem Oberbegriff des Anspruchs 1.

Es ist bekannt, zur Eröffnung des Eintrittspunktes z. B. eines proximal einzubringenden Femurnagels zunächst einen Führungsdraht in den Eintrittspunkt einzubringen. Anschließend wird mit einem über den Draht gefädelten Bohrer der Knochen geöffnet. Ein Vorteil dieser Technik besteht darin, daß vor dem eigentlichen Bohren mit einem relativ großen Durchmesser die genaue Lage und Richtung des Führungsdrahtes und damit auch der anschließenden Bohrung mit Hilfe eines Röntgengerätes überprüft werden kann.

Zur Vermeidung von Weichteilverletzungen durch den Bohrer ist auch bekannt, zuerst eine Gewebeschutzhülse über den Führungsdraht zu schieben, bis sie am Knochen aufliegt. Es ist ferner bekannt, die Gewebeschutzhülse mit einem Trokar zu verschließen, um ein besseres Hindurchgleiten durch die Weichteile zu ermöglichen. Anschließend wird das Trokar vor dem Bohrvorgang entfernt.

Aus US 5,951,561 ist ein Trokar bekannt geworden, das eine Mehrzahl von achsparallelen Durchbohrungen aufweist. Das Trokar ist in der Gewebeschutzhülse drehbar gelagert. Wird bei der Röntgenkontrolle festgestellt, daß z. B. die mittige Bohrung im Trokar zur gewünschten Lage seitlich versetzt liegt, wird in einem anderen geeigneten Loch ein zweiter Führungsdraht gesetzt. Liegt der zweite Draht an der gewünschten Stelle, wird der erste Draht entfernt und der zweite Draht überbohrt.

Der Erfindung liegt die Aufgabe zugrunde, eine Vorrichtung zum lagerichtigen Einbringen eines Führungsdrahtes für ein Bohrwerkzeug in einen Knochen zu schaffen, mit der die Handhabbarkeit und die Genauigkeit zum Setzen des Führungsdrahtes verbessert wird.

Diese Aufgabe wird durch die Merkmale des Anspruchs 1 gelöst.

Bei der Erfindung ist ein zweiter Griff vorgesehen, der sich annähernd in die gleiche Richtung wie der erste Griff erstreckt und dessen eines Ende so außerhalb der Schutzhülse am Trokar angreift, daß das normalerweise frei drehbare Trokar gegenüber der Schutzhülse in Drehrichtung festgesetzt wird, wenn beide Griffe von Hand gegeneinander gespannt werden.

Die beiden Griffe sind derart angeordnet, daß sie zusammen von einer Hand erfaßt werden können. Die Verbindung mit dem Trokar ist nun derart, daß bei einem Zusammenspannen beider Griffe das Trokar in seiner Drehlage festgelegt wird.

Es sind verschiedene konstruktive Möglichkeiten zum Festsetzen des Trokars denkbar. Eine besteht nach einer Ausgestaltung der Erfindung darin, daß das Trokar eine Ringausnehmung aufweist, in der ein ringförmiger Lagerabschnitt des zweiten Griffes so eingreift, daß der ringförmige Lagerabschnitt in der Ausnehmung verkantet und das Trokar klemmend erfaßt, wenn die Griffe gegeneinander gespannt werden. Bei einem operativen Eingriff wird, falls erforderlich, der zweite Führungsdraht in eine andere Bohrung eingeführt, welche der gewünschten Position näher kommt. Durch die klemmende Festlegung des Trokars in Drehrichtung wird vermieden, daß durch eine geringfügige Relativdrehung des Trokars zusammen mit dem ersten Führungsdraht der zweite Führungsdraht eine falsche Richtung wählt und nicht die gewünschte Lage erreicht.

Vor dem operativen Eingriff wird die gewünschte Drehlage des Trokars eingestellt. Hierzu weist nach einer Ausgestaltung der Erfindung der Abschnitt des Trokars an dem der Schutzhülse abgewandten Ende einen Drehknopf auf.

Der zweite Griff ist vorzugsweise aus einem elastischen Material, so daß er zum ersten Griff hin gebogen werden kann, wenn beide Griffe zusammengespannt werden. Vorzugsweise liegt der zweite Griff oberhalb des ersten Griffes.

Um eine möglichst raumsparende Griffanordnung zu erzielen, sieht eine Ausgestaltung der Erfindung vor, daß der zweite Griff einen Längsschlitz aufweist sowie eine zum ersten Griff hin weisende Durchbiegung oder Abknickung derart, daß ein Abschnitt des ersten Griffes in den Längsschlitz eingreift. Es versteht sich, daß auch der erste Griff einen Abschnitt aufweisen kann, der zum zweiten Griff hin gebogen sein kann und annähernd passend in den Schlitz eingreift.

Nach einer anderen Ausgestaltung der Erfindung weist der Schaft des Trokars mehrere in Achsrichtung im Abstand angeordnete radiale Bunde auf, deren Außendurchmesser etwas kleiner ist als der Innendurchmesser der Gewebeschutzhülse, und ein Teil der axialen Bohrungen in den Bunden setzt sich zwischen den Bunden als achsparallele Nuten fort. Bei dieser Ausführungsform ist nicht erforderlich, daß das Trokar über seine gesamte Länge gleiche Dicke aufweist mit mehreren Durchbohrungen. Letzere befinden sich nur in den Bunden, während zwischen den Bunden achsparallele Nuten oder Führungskanäle geformt sind. Dies gilt zumindest für einen Teil der mehreren Bohrungen.

Ein Ausführungsbeispiel der Erfindung wird nachfolgend anhand von Zeichnungen näher erläutert.
- Fig. 1: zeigt eine Vorrichtung nach der Erfindung in perspektivischer Darstellung im auseinandergebauten Zustand.
- Fig. 2: zeigt perspektivisch die Vorrichtung nach Fig. 1 im zusammengebauten Zustand.
- Fig. 3: zeigt die Endansicht des Trokars der Vorrichtung nach den Fign. 1 und 2.

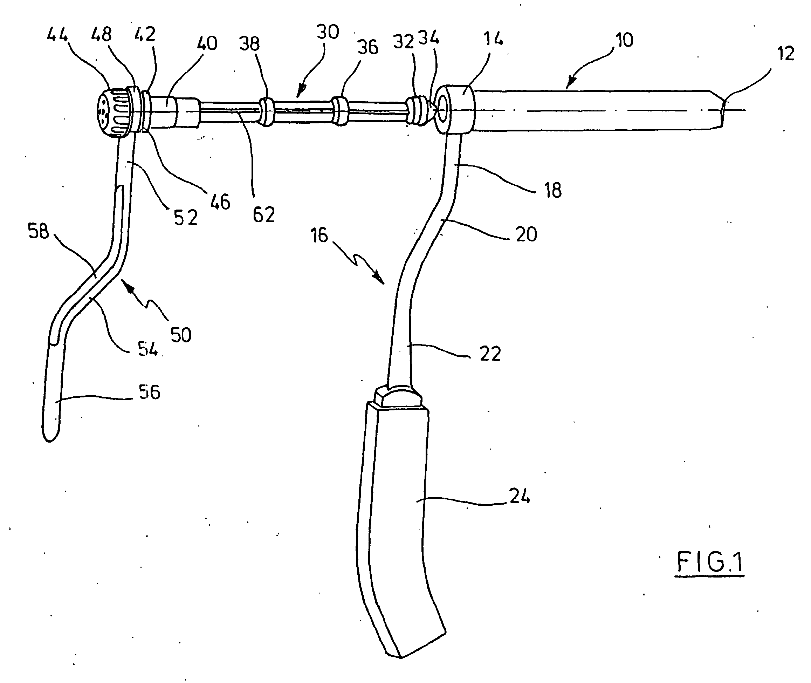

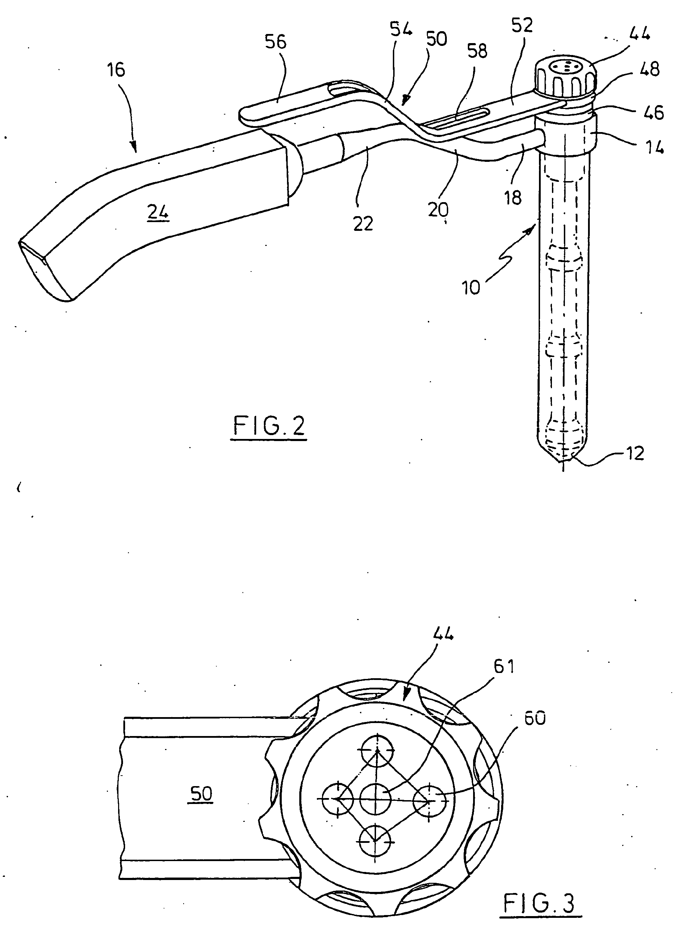

Die in den Fign. 1 und 2 dargestellte Vorrichtung weist eine Gewebeschutzhülse 10 auf mit einem rechten Ende, das als eine verhältnismäßig scharfe unregelmäßige Kante 12 ausgebildet ist und einem verdickten linken Ende 14, an dem ein Griff 16 radial abstehend angebracht ist. Der Griff ist in einem ersten Abschnitt stangenartig geformt mit einem ersten Abschnitt 18 am verdickten Ende 14, einem schräg nach oben gebogenen Abschnitt 20 und einem wiederum annähernd in die gleiche Richtung wie der Abschnitt 18 weisenden dritten Abschnitt 22. Die Abschnitte 18 bis 22 sind annähernd kreisförmig im Querschnitt. Daran schließt sich ein Greifabschnitt 24 an, der z. B. aus Kunststoff bestehen kann und auf den Abschnitt 22 aufgeschoben ist, wobei die Abschnitte 18 bis 22 aus einem Metall geformt sein können.

In Fig. 1 ist ferner ein Trokar 30 zu erkennen, das ein stopfenförmiges rechtes Ende 32 aufweist mit einer gerundeten Spitze 34. Außerdem sind am Schaft im Achsabstand zwei radiale Bunde 36, 38 vorgesehen, wobei der Schaft zwischen den Bunden 36, 38 bzw. Bund 36 und dem Stopfenabschnitt 32 einen kleineren Durchmesser aufweist. Der Außendurchmesser der Bunde 36, 38 und des Stopfenabschnitts 32 ist derart, daß er geringfügig kleiner ist als der Innendurchmesser der Gewebeschutzhülse 10. Am linken Ende weist der Schaft einen Endabschnitt 40 auf, der wiederum den Außendurchmesser der Bunde 36, 38 aufweist. An den Abschnitt 40 schließt sich ein im Durchmesser größerer Flansch 42 an und am Ende des Schaftes ist ein Drehknopf 44 geformt. Zwischen Drehknopf 44 und Flansch 42 ist eine ringförmige Ausnehmung 46 geformt. In der ringförmigen Ausnehmung 46 sitzt ein ringförmiger Abschnitt 48 eines zweiten Griffes 50. Der ringförmige Abschnitt 48 ist geringfügig in der ringförmigen Ausnehmung 46 axial beweglich und kippbar.

Der zweite Griff 50 weist einen flachen ersten Griffabschnitt 52 auf, der annähernd radial von dem Schaft des Trokars 30 absteht. Ferner sieht er einen abgebogenen Abschnitt 54 vor sowie einen wiederum annähernd radial sich erstreckenden Abschnitt 56. In den Abschnitten 52 und 54 ist ein Längsschlitz 58 von gleicher Breite geformt.

Beim Zusammenbau wird der Schaft des Trokars 30 in die Gewebeschutzhülse 10 eingeführt, wobei sich der Flansch 42 gegen die Stirnseite des linken Endes der Gewebeschutzhülse 10 anlegt. Die Griffe sind übereinander angeordnet, und der abgebogene Griffabschnitt 20 greift annähernd passend in den Längsschlitz 58 des zweiten Griffes 50. Dadurch ist die Drehlage der Griffe 16, 50 zueinander bzw. vor Griff 50 und Gewebeschutzhülse 10 festgelegt.

Wie insbesondere aus Fig. 3 hervorgeht, weist das Trokar 30 fünf axiale parallele Bohrungen 60 auf, die sich bis zum Endabschnitt 32 des Trokars 30 erstrecken und zur Aufnahme eines Führungsdrahtes (nicht gezeigt) zum Aufbohren von Knochen gedacht sind. Wie aus Fig. 1 zu erkennen, ist im massiven Teil des Schaftes des Trokars 30 eine zentrale Bohrung 61 vorgesehen, während die anderen Bohrungen zwischen den Bunden 36, 38 und dem stopfenförmigen Ende 32 als achsparallele Nuten geformt sind, wie bei 62 in Fig. 1 zu erkennen.

Die Drehlage des Trokars 30 innerhalb der Gewebeschutzhülse 10 kann durch den Chirurgen durch Drehung am Knopf 44 eingestellt werden. Durch Ergreifen beider Griffe 16, 50 von Hand kann die Drehlage des Trokars 30 festgelegt werden, indem der ringförmige Abschnitt 48 in der ringförmigen Ausnehmung 46 verkantet und dadurch das Trokar 30 festklemmt. Wie erkennbar ist der Griff 50 kürzer als der Griff 16. Dadurch wird das Halten der Gewebeschutzhülse 10 ohne Betätigung des Griffes 50 möglich.

Wird bei der Operation festgestellt, daß der in die mittige Bohrung 61 eingeführte Führungsdraht bei der Röntgenbetrachtung nicht die gewünschte Lage hat, wird nach Drehung des Trokars 30 ein zweiter Führungsdraht durch eine der Bohrungen 60 eingeführt. Hat der zweite Führungsdraht die richtige Position, wird der erste Führungsdraht entfernt. Nach dem vollständigen Einführen des Führungsdrahtes wird das Trokar aus der Gewebeschutzhülse 10 entfernt, und mit Hilfe eines Bohrwerkzeugs kann nunmehr, auf den Führungsdraht aufgefädelt, das Aufbohren des Knochens vor sich gehen.

## Patentansprüche

1. Vorrichtung zum lagerichtigen Einbringen eines Führungsdrahtes für ein Bohrwerkzeug in einen Knochen mit einer Gewebeschutzhülse (10), die an einem Ende einen seitlich abstehenden Griff (16) aufweist, und einem Trokar (30), das vom Griffende her in die Gewebeschutzhülse einsetzbar ist und deren anderes Ende verschließt, wobei das in der Gewebeschutzhülse drehbare Trokar eine zentrale Bohrung (61) und mehrere exzentrische achsparallele Bohrungen (60) aufweist, durch welche ein Führungsdraht hindurch geführt werden kann, **dadurch gekennzeichnet, daß** ein zweiter Griff (50) vorgesehen ist, der sich annähernd in die gleiche Richtung wie der erste Griff (16) erstreckt und dessen eines Ende so am außerhalb der Gewebeschutzhülse (10) liegenden Ende des Trokars (30) angreift, daß das normalerweise frei drehbare Trokar (30) gegenüber der Gewebeschutzhülse (10) in Drehrichtung festgesetzt wird, wenn beide Griffe (16, 50) von Hand gegeneinander gespannt werden.

2. Vorrichtung nach Anspruch 1, **dadurch gekennzeichnet, daß** das Trokar (30) eine ringförmige Ausnehmung (46) aufweist, in der ein ringförmiger Lagerabschnitt (48) des zweiten Griffes (50) so eingreift, daß der ringförmige Lagerabschnitt (48) in der Ausnehmung (46) verkantet und das Trokar (30) klemmend erfaßt, wenn die Griffe (16, 50) gegeneinander gespannt werden.

3. Vorrichtung nach Anspruch 2, **dadurch gekennzeichnet, daß** der Abschnitt des Trokars (30) an dem der Gewebeschutzhülse abgewandten Ende als Drehknopf (44) ausgebildet ist.

4. Vorrichtung nach einem der Ansprüche 1 bis 3, **dadurch gekennzeichnet, daß** der Schaft des Trokars (30) mehrere in Achsrichtung im Abstand angeordnete radiale Bunde (36, 38) aufweist, deren Außendurchmesser etwas kleiner ist als der Innendurchmesser der Gewebeschutzhülse (10) und ein Teil der axialen Bohrungen (40) in den Bunden (36, 38) sich als achsparallele Nuten (62) zwischen den Bunden (36, 38) fortsetzen, die in Abschnitten mit kleinerem Durchmesser geformt sind.

5. Vorrichtung nach einem der Ansprüche 1 bis 4, **dadurch gekennzeichnet, daß** der zweite Griff (50) aus elastisch verbiegbarem Material besteht.

6. Vorrichtung nach einem der Ansprüche 1 bis 5, **dadurch gekennzeichnet, daß** der zweite Griff (50) oberhalb des ersten Griffes (16) angeordnet ist.

7. Vorrichtung nach Anspruch 6, **dadurch gekennzeichnet, daß** der zweite Griff (50) einen Längsschlitz (58) aufweist sowie einen zum ersten Griff (16) hin weisenden Abschnitt (54) derart, daß ein Abschnitt (20) des ersten Griffes (16) in den Längsschlitz (58) eingreift.

## Claims

1. A device for the insertion, in the correct position, of a guide wire for a drilling tool in a bone with a tissue protection sleeve (10) which has at one end a laterally projecting handle (16), and with a trocar (30) which can be inserted from the gripping end into the tissue protection sleeve and is sealing its other end, wherein the trocar that can be rotated in the tissue protection sleeve has a central hole (61) and a plurality of eccentric parallax holes (60) through which a guide wire can be guided, **characterised in that** a second handle (50) is provided which extends approximately in the same direction as the first handle (16) and one of whose ends engages on the end of the trocar (30) located outside the tissue protection sleeve (10) so that the normally freely rotatable trocar (30) is fixed in its direction of rotation relative to the tissue protection sleeve (10) when both handles (16, 50) are clamped together by hand.

2. The device according to Claim 1, **characterised in that** the trocar (30) has an annular recess (46) in which an annular bearing section (48) of the second handle (50) engages so that the annular bearing section (48) tilts in the recess (46) and so that the trocar (30) is gripped in a clamping manner when the handles (16, 50) are clamped against each other.

3. The device according to Claim 2, **characterised in that** the section of the trocar (30) on the end facing away from the tissue protection sleeve is formed as a rotary knob (44).

4. The device according to one of Claims 1 to 3, **characterised in that** the shaft of the trocar (30) has a plurality of radial collars (36, 38) arranged so that they are spaced apart in the axial direction, the outside diameter of which collars is slightly smaller than the inside diameter of the tissue protection sleeve (10), and **in that** some of the axial holes (40) in the collars (36, 38) continue as parallax grooves (62) between the collars (36, 38), which are formed in sections with a smaller diameter.

5. The device according to one of Claims 1 to 4, **characterised in that** the second handle (50) consists of elastically bendable material.

6. The device according to one of Claims 1 to 5, **characterised in that** the second handle (50) is arranged above the first handle (16).

7. The device according to Claim 6, **characterised in that** the second handle (50) has a longitudinal slot (58) and a section (54) pointing towards the first handle (16) so that a section (20) of the first handle (16) engages in the longitudinal slot (58).

## Revendications

1. Dispositif destiné à la mise en place en bonne position d'un fil-guide pour un outil de perçage dans un os, comportant une canule de protection de tissus (10) qui à une extrémité comporte une poignée (16) faisant saillie latéralement et un trocart (30) qui peut être inséré dans la canule de protection de tissus à partir de la poignée et ferme son autre extrémité, le trocart étant capable de tourner dans la canule de protection de tissus comportant un perçage central (61) et plusieurs perçages (60) excentrés d'axes parallèles à travers lesquels peut être inséré un fil-guide, **caractérisé en ce qu'**il est prévu une seconde poignée (50), qui s'étend approximativement dans la même direction que la première poignée (16) et dont une extrémité vient en prise sur l'extrémité du trocart (30) situé à l'extérieur de la canule de protection de tissus (10) de telle façon que le trocart (30), normalement capable de tourner librement, est immobilisé en rotation par rapport à la canule de protection de tissus (10) lorsque les deux poignées (16, 50) sont pressées l'une contre l'autre par une main.

2. Dispositif selon la revendication 1, **caractérisé en ce que** le trocart (30) comporte un évidement annulaire (46) dans lequel une section de palier annulaire (48) de la seconde poignée (50) vient en prise de telle façon que la section de palier annulaire (48) se bloque dans l'évidement (46) et saisit le trocart (30) à la manière d'une pince lorsque les poignées (16, 50) sont pressées l'une contre l'autre.

3. Dispositif selon la revendication 2, **caractérisé en ce que** la section du trocart (30) est réalisée à l'extrémité opposée de la canule de protection de tissus, sous forme de bouton rotatif (44).

4. Dispositif selon l'une quelconque des revendications 1 à 3, **caractérisé en ce que** la tige du trocart (30) comporte plusieurs collerettes (36, 38) disposées à distance dans la direction axiale, dont le diamètre extérieur est légèrement inférieur au diamètre intérieur de la canule de protection de tissus (10) et qu'une partie des perçages axiaux (40) dans les collerettes (36, 38) se poursuivent sous forme de rainures d'axes parallèles (62) entre les collerettes (36, 38) qui sont formées dans des sections de plus petit diamètre.

5. Dispositif selon l'une quelconque des revendications 1 à 4, **caractérisé en ce que** la seconde poignée (50) est réalisée en matériau flexible élastiquement.

6. Dispositif selon l'une quelconque des revendications 1 à 5, **caractérisé en ce que** la seconde poignée (50) est disposée au-dessus de la première poignée (16).

7. Dispositif selon la revendication 6, **caractérisé en ce que** la seconde poignée (50) comporte une fente longitudinale (58) ainsi qu'une section (54) orientée vers la première poignée (16) telle qu'une section (20) de la première poignée (16) vient en prise dans la fente longitudinale (58).
